# EUROPEAN PATENT APPLICATION

(11) **EP 4 235 687 A1**
(43) Date of publication of application: **30.08.2023**
(21) Application number: 22159112.6
(22) Date of filing: 28.02.2022
(51) Int. Cl.: G16H 40/20, G16H 40/40, G16H 40/60, G16H 40/63, G16H 40/67

(54) **APPARATUS, METHOD AND SYSTEM FOR ASSIGNING PROCESSING STEPS TO A LABORATORY AUTOMATON SYSTEM**

(71) Applicant: Beckman Coulter, Inc., Brea, CA 92821 (US)
(72) Inventor: VARLET, Eric, 59790 Ronchin (FR); REICHLE, Roland, 80689 München (DE)
(74) Representative: Müller-Boré & Partner Patentanwälte PartG mbB

(57) **Abstract**

A computer-implemented method is provided. The method comprises: accessing, by a first computing device, sample-processing data, wherein the sample-processing data comprise information indicative of a set of procedures to be carried out on a biological sample, wherein each procedure comprises one or more actions; obtaining, by the first computing device, ALS1 state data, wherein the ALS1 state data comprise information indicative of a state of a first automated laboratory system - ALS1; obtaining, by the first computing device, ALS2 operation data, wherein the ALS2 operation data comprise information indicative of an ALS2 set of actions of at least a first procedure, wherein the ALS2 set of actions either has been carried out or is scheduled to be carried out by a second automated laboratory system - ALS2 - on the biological sample; generating, by the first computing device, ALS1 processing data by using the sample-processing data, the ALS1 state data and the ALS2 operation data, wherein the ALS1 processing data comprise information indicative of an ALS1 set of actions of at least a second procedure of the sets of procedures; and initiating, by the first computing device, the generation of an ALS1 route plan for the first automated laboratory system, the ALS1 route plan being based on the ALS1 processing data.

## Description

### Technical Field

The present invention relates to the field of biological samples testing.

### Background

Analytic and clinical laboratories typically comprise several laboratory instruments that are integrated in an automated laboratory system and are configured to automatically carry out one or more clinical tests on biological samples (such as blood samples).

### Summary

It is an object of the invention to provide means for improving the reliability and efficiency of the processing of biological samples for clinical test.

The achievement of this object in accordance with the invention is set out in the independent claims. Further developments of the invention are the subject matter of the dependent claims.

According to a first aspect, a computer-implemented method is provided. The method comprises:
- accessing, by a first computing device, sample-processing data, wherein the sample-processing data comprise information indicative of a set of procedures to be carried out on a biological sample, wherein each procedure comprises one or more actions;
- obtaining, by the first computing device, ALS1 state data, wherein the ALS1 state data comprise information indicative of a state of a first automated laboratory system (hereinafter also referred to as:"ALS1" or "first ALS");
- obtaining, by the first computing device, ALS2 operation data, wherein the ALS2 operation data comprise information indicative of an ALS2 set of actions of at least a first procedure, wherein the ALS2 set of actions either has been carried out or is scheduled to be carried out by a second automated laboratory system (hereinafter also referred to as:"ALS2" or "second ALS") on the biological sample;
- generating, by the first computing device, ALS1 processing data by using the sample-processing data, the ALS1 state data and the ALS2 operation data, wherein the ALS1 processing data comprise information indicative of an ALS1 set of actions of at least a second procedure of the set of procedures; and
- initiating, by the first computing device, the generation of an ALS1 route plan for the first automated laboratory system, the ALS1 route plan being based on the ALS1 processing data.

A computing device, e.g. the first computing device, may comprise at least one memory and at least one processor. A computing device may also comprise one or more input/output units.

In the present disclosure, "accessing, by a computing device (e.g. the first computing device), data" may comprise retrieving the data e.g. from the at least one memory of said computing device, from the memory of another computing device, or from another remote data storage (a database, a secondary memory, a cloud storage or the like). Accordingly, in some cases, retrieving data may comprise downloading data. Exemplarily, said computing device may retrieve data in response to a user command and/or to a notification sent by another computing device.

Additionally or alternatively, "accessing, by a computing device, data" may comprise receiving the data, e.g. from a user or another computing device. The two options are not mutually exclusive. For instance, accessing, by a computing device, data may comprise receiving the data, storing the data in the memory of said computer device and retrieving the data by accessing said memory.

The first computing device accesses sample-processing data, the sample-processing data comprising information indicative of a set of procedures to be carried out on a biological sample. The biological sample (or simply "sample") may be a sample of a bodily fluid of a human or animal subject. For example, the bodily fluid may be a physiological fluid, such as blood, saliva, urine, sweat, amniotic fluid, cerebrospinal fluid, ascites fluid, or the like.

The biological sample may be put into a sample container after collection and it may be held in the container while being processed. The sample container may be a sample tube. Typically, sample tubes comprise a closed tube end and an end opposite thereto. The latter end defines an opening for inserting the sample in the sample tube. The opening may be closed, e.g. sealed, by a cap. In addition to the biological sample, the sample container may contain one or more substances such as reagents.

Generally, a set may comprise one or more elements. Accordingly, the set of procedures may comprise one or more procedures and a set of actions may comprise one or more actions.

The sample-processing data comprise information specifying a set of procedures to be carried out on the biological sample. A procedure comprises one or more actions, e.g. a sequence of actions that shall be performed on the biological sample and/or on the sample container to accomplish a task and/or achieve a result. For example, a procedure may be a clinical test. A clinical test allows for accomplishing a task, namely the task of estimating the value of a parameter concerning the sample, e.g. a clinical parameter.

In particular, according to the present invention, an action may be a laboratory operation, such as capping or decapping a sample tube, centrifuging or analysing the biological sample.

In some examples, the information comprised in the sample-processing data may denote the procedure as a whole without providing an indication of the single action(s) involved in the procedure. In the example of the blood test for estimating a clinical parameter X, the sample-processing data may comprise information indicative of the fact that a blood test for clinical parameter X is to be carried out, but not of the centrifuging, reagent addition and analysis.

In this case, for instance, the first computing device may obtain the set of actions comprised in the procedure. In particular, the sample-processing data may comprise an alphanumeric string that uniquely specifies the procedure. For example, the first computing device may use the aforementioned alphanumeric string to retrieve information indicative of the set of actions comprised in the procedure. The latter information may be a list of alphanumeric strings, each string of said list being uniquely associated with a respective action.

Alternatively or in conjunction with the above, the information comprised in the sample-processing data may provide an explicit indication of the actions comprised in the procedure. Accordingly, the sample-processing data may comprise information indicative of the set of actions comprised in the procedure and/or of each actions of said set of actions. For example, the sample-processing data comprises a set of alphanumeric strings, each alphanumeric string of said list being uniquely associated with and uniquely specifying a respective action of the set of actions comprised in the procedure.

The information indicative of a particular procedure to be carried out on the sample may comprise an indication of which procedure is to be performed (e.g. which kind of clinical test) and, optionally, an indication of a timing constraint on the procedure (e.g. expressed as due date and/or priority). Furthermore, in some examples, the information indicative of a particular procedure to be carried out on the sample may comprise an indication of one or more features of the sample (e.g. sample volume or sample type such as blood) and/or of the sample container (e.g. tube type).

Generally, data in this disclosure may comprise information encoded in human-readable format and/or in machine-readable format. In the case of the sample-processing data, the human-readable format may comprise text in a natural language and/or alphanumeric strings that denote a specific procedure/action, wherein the relation between an alphanumeric string and the corresponding procedure/action may be collected in a dictionary, the dictionary being stored in a memory device, e.g. in a database accessible by the first computing device, by a computing device of the ALS1 and/or by a computing device of the ALS2.

In particular, a procedure may be carried out by an automated laboratory system configured to carry out each action of the set of actions comprised in the procedure. For example, an action may be carried out by an automated laboratory system configured to perform, by using its laboratory components, a set of processing steps, e.g. a chronological sequence of processing steps, to complete the action. In particular, the set of processing steps defines a process that, when performed by the automated laboratory system, leads to the completion of the action. In general, the set of processing steps depends on the automated laboratory system that carries out the action, e.g. on the configuration thereof. In particular, different automated laboratory systems may have different laboratory components or laboratory components configured differently and, hence, may perform different processing steps for carrying out an action.

A processing step is performed by a laboratory component, such as an instrument, on the biological sample. The processing step can be carried out by directly interacting with the sample (e.g. aliquoting) and/or by indirectly interacting with the sample (e.g. transporting the sample container containing the sample). The interaction may be mechanical, chemical, biological, optical or of another kind, and it may involve a combination of two or more kinds. In some examples, a processing step may be non-invasive and may leave the sample substantially unaltered, e.g. a processing step may move the sample container from one component of an automated laboratory system to another component of said automated laboratory system. A processing step may gather information from the sample via a substantially non-invasive technique (such as spectroscopy).

In particular, information indicative of an action may specify the action without reference to how the procedure is actually carried out in practice by using the laboratory components of a particular automated laboratory instrument (e.g. ALS1 or ALS2). Accordingly, if the sample-processing data specifies a particular procedure by its set of actions, the sample-processing data do not need to comprise, for the actions of said set of actions, information indicative of the set of processing steps that, when carried out by an automated laboratory instrument (e.g. ALS1 or ALS2), leads to the completion of the actions of said set of actions.

For example, the procedure may be a blood test for estimating a clinical parameter X, which comprises the following actions: centrifuging the sample, adding a reagent to the sample, analysing the sample. These instructions do not refer to instruments for carrying out each action, nor indicate any details on how to perform the action (e.g. how much reagent), nor consider supplementary or intermediate steps bridging the actions, such as moving the sample from one laboratory component to another.

Accordingly, a procedure as a whole may affect the sample by modifying one or more of its features, such as its position, orientation, volume, composition, physical state, chemical state and so on. Summarising, the procedure comprises one or more actions and the execution of the procedure comprises one or more processing steps, in that the implementation of each action within an automated laboratory instrument comprises performing one or more processing steps. By extension, it may be said that the procedure comprises one or more processing steps.

The method further comprises obtaining, by the first computing device, ALS1 state data, the ALS1 state data comprising information indicative of a state of a first ALS, as well as obtaining, by the first computing device, ALS2 operation data, wherein the ALS2 operation data comprise information indicative of an ALS2 set of actions of at least a first procedure, wherein the ALS2 set of actions either has been carried out or is scheduled to be carried out by a second automated laboratory system on the biological sample.

Generally, an automated laboratory system (hereinafter also referred to as: "ALS") is an assembly comprising a plurality of components and a computing device, wherein the computing device is operatively connected to these components and is configured to control each component. A component may be an analytic instrument, a pre-analytic instrument, a post-analytic instrument, an input/output module, a transportation component (e.g. track, belt, tube carrier) configured to move a sample.

An analytic instrument (e.g. analyser) is an instrument configured to carry out one or more analytic steps, such as measuring one or more characteristics of the sample, e.g. the concentration of a given analyte. A pre-analytic instrument is an instrument configured to carry out one or more pre-analytic steps on a biological sample to prepare said sample for the analytic instrument(s). For example, centrifuges, aliquoters and de-cappers are pre-analytic instruments. A post-analytic instrument is an instrument configured to carry out one or more post-analytic steps on the biological sample after the sample has been processed by one or more analytic instruments. For example, re-cappers and sample storage units are post-analytic instruments. The term "instrument" will be used to generally refer to an analytic instrument, a pre-analytic instrument or a post-analytic instrument.

Generally, an ALS may comprise one or more subsystems, wherein a subsystem comprises one or more components of the ALS. In particular, the subsystem may coincide with the ALS or the subsystem may comprise a subset of the components of ALS. Exemplarily, (a subsystem of) an ALS may be configured to carry out a plurality of processing steps on a biological sample. In particular, processing steps that can be performed on a sample (e.g. those required by the procedure(s) of the sample-processing data) may comprise any (or combinations of) pre-analytic steps, post-analytic steps, analytic steps.

The method comprises obtaining ALS1 state data and ALS2 operation data. In the present disclosure, "obtaining, by a computing device (e.g. the first computing device), data" may comprise accessing the data as discussed above.

Alternatively, "obtaining, by a computing device, data" may comprise generating the data, i.e. creating the data based on one or more inputs. For instance, an input may be information from an ALS component, e.g. in case the computing device is part of the ALS, as discussed below.

In yet another example, "obtaining, by a computing device, data" may comprise accessing a first portion of the data and generating a second portion of the data from the first portion of the data.

Generally, state data comprising information indicative of a state of an ALS comprise information indicative of a state of at least a subsystem of an ALS, namely of a current or expected state. In other words, the state data comprise information specifying a current or expected state of at least a subsystem of the ALS, i.e. of one or more subsystems. The state of an ALS (or a subsystem thereof) may be defined by one or more aspects of the ALS (or the subsystem), e.g. including the states and/or functionalities of the individual components. In the following, the state of the ALS is discussed, but the same applies for only one or more subsystems of the ALS.

Exemplarily, the state data comprise information indicative of the suitability of the ALS to carry out a given procedure/action. The information may be provided at a coarse level, e.g. for the ALS as a whole and not the specific components. In other words, while the suitability of the ALS may exemplarily be determined based on the single components, the information indicative of the suitability (as comprised in the state data) may specify the suitability of the ALS as a whole, without disclosing the suitability of the single components.

In particular, the suitability of the ALS may be determined by its capability of performing the procedure/action and/or its availability to process a sample in view of the current and/or expected workload at the ALS.

With regards to the capability, it may be based on any of the following or combinations thereof: whether the ALS comprises instruments that can carry out a given procedure and/or given actions, whether such instruments are currently functioning or not, whether the ALS is capable of handling a given type of container, performance characteristics of the ALS (and in particular of its components), such as speed or accuracy of analysis, wherein the accuracy may be determined by a quality control (QC) status or trend.

With regards to the availability, the workload may be determined based at least on 1) a number of samples being processed by the ALS at a given timepoint and, optionally, further based on 2) a forecasted leftover time for each of these samples. In particular, the number of samples being processed by the ALS (also referred to as "being onboard the ALS") may be a relative quantity considered with respect to a capacity of the ALS. For instance, the capacity of the ALS may be given by the number of samples that can be accommodated in the input module and/or the output module of the ALS. Accordingly, the number of samples onboard the ALS may be expressed as a percentage.

In particular, the current workload may be determined based on the number of samples that are being processed by the ALS at the moment in which the state data are accessed (e.g. the workload may be determined on-the-fly) and, optionally, their forecasted leftover time.

The expected workload may be determined based on the number of samples that are anticipated to be processed by the ALS at the moment in which the sample is expected to arrive at the ALS (or "expected arrival time"). The anticipated number of samples may be extracted from a schedule of the ALS, which may be accessed by the first computing device. The expected arrival time of the sample at the ALS may be a piece of data that is accessed by the first computing device, e.g. together with the first sample-processing data.

For example, the ALS may be considered available if the number of samples being processed at the relevant timepoint is lower than a (first) predetermined threshold, which may e.g. be equal to the maximum number of samples that can be processed at the same time by the ALS. In particular, the availability may be considered within a time window, so that the first predetermined threshold may be given as a number of samples per time window e.g. 10 samples per 30 seconds. Additionally, the ALS may also be considered available if the number of samples being processed at the relevant timepoint is equal to the (first) predetermined threshold and the forecasted leftover time of at least one of the samples being processed is lower than a (second) predetermined threshold, e.g. half of the time window.

The availability may be based on a current workload if the sample is already ready to be transported to the ALS and the transport time of the sample to the ALS is lower or equal than a (third) predetermined threshold (e.g. 2 minutes), while it may be based on an expected workload otherwise. The transport time may be estimated based on sample location data comprising information indicative of a location of the sample, which may be accessed by the first computing device.

In some examples, the first computing device may receive the information indicative of the suitability (in particular, the availability) of the ALS, i.e. (at least part of) the state data, from the corresponding ALS. In other examples, the first computing device may compute/predict (e.g. via a simulation) the availability on the basis of data received from the ALS (such as number of samples being processed) optionally combined with other data received from other sources (such as an expected arrival time or a location of the sample).

In a particular example, the state data of an ALS may further comprise information indicative of a state of a reagent of an instrument of the ALS, information indicative of a calibration state of an instrument, information indicative of a quality control state of an instrument of the ALS, information indicative of a configuration of an instrument, information indicative of an arrangement of the components of the ALS with respect to one another, and/or information indicative of a state of a component of the ALS.

The state of a reagent of an instrument may specify whether the reagent is present or not and, in the affirmative case, how much reagent is present. The calibration state of an instrument of the ALS may refer to the calibration state for a test carried out by the instrument by using a respective reagent, e.g. from a reagent lot. The quality control state may specify that the instrument is currently unsuitable for carrying out a particular test in a reliable way.

The configuration of an instrument may refer to the settings of the instrument, e.g. the values of parameters that control how the instrument performs a certain operation, and/or to the functionalities of the instrument, e.g. which actions/procedures the instrument is configured to carry out. The arrangement of the components of the subsystem with respect to one another may specify the spatial relations between the component. The state of a component may specify whether the component is down / under maintenance / available. It may also comprise information about the workload of the component.

Accordingly, in some examples, the state data may take into account planned QC procedures to be run on, planned calibration of, or other planned maintenance procedures to be run the instruments of the ALS.

In addition to the ALS1 state data, the first computing device obtains the ALS2 operation data comprising information indicative of an ALS2 set of actions that either has been carried out or is scheduled to be carried out by a second ALS2 on the biological sample.

The second ALS is not the same as the first ALS. The ALS1 and the ALS2 may be at two locations physically separated from each other. The two locations may be within a limited area, such that the computing devices of the ALS1 and of the ALS2 may be interconnected by a local area network (LAN). Alternatively, the two locations may be remote with respect to each other and their respective computing devices may be connected by a wide area network (WAN), such as the Internet.

Exemplarily, the ALS1 and the ALS2 may at least partially comprise different components, such as different analytic instruments and or analytic instruments with different configurations. For instance, a first component in the ALS1 may differ from a second component in the ALS2 in that, even if they are both configured to perform the same processing step, they are configured to do it in a different manner, e.g. using different parameters. Alternatively or additionally, the ALS1 (or ALS2) may comprise at least one component (e.g. an analytic instrument) that is configured to perform a processing step and the ALS2 (or ALS1) may not comprise any component configured to perform said processing step. In other examples, the ALS1 and the ALS2 may comprise identical components. In particular, the ALS1 and ALS2 may comprise identical components which may or may not be configured in the same way.

The first ALS may comprise the first computing device. Alternatively, the first computing device may be comprised in the second ALS. In yet another example, the first computing device may neither be part of the ALS1 nor of the ALS2. If a computing device "receives" data from the ALS it belongs to (as discussed e.g. above with regards to the state data), it may retrieve the data from its own memory and/or access the data from the components of the ALS.

Exemplarily, the first ALS (in particular a subsystem thereof) may be configured to carry out at least a second procedure of the set of procedures. The second ALS (in particular a subsystem thereof) may be configured to carry out at least a first procedure (which may optionally be part of the set of procedures), the second procedure being different from the first procedure. In other words, exemplarily, the set of procedures of the sample-processing data may comprise a second procedure to be carried out by the ALS1 and a first procedure to be carried out by the ALS2. The first procedure and the second procedure may have an overlap, i.e. there may be at least one action that is common to both procedures.

The biological sample may be processed by both the ALS1 and the ALS2, wherein the ALS2 may process the sample before the ALS1 processes the sample, e.g. according to a predetermined scheduling order, which may be fixed or variable. For example, in the latter case, there may be a plurality of ALSs and the first computing device may determine which specific ALSs and in which order they should process the sample, i.e. carry out procedures on the sample. Exemplarily, the first computing device may choose an ALS (e.g. the ALS2) to carry out a given procedure on the sample before another ALS (e.g. the ALS1) carries out another procedure.

For instance, in some examples, the method may further comprise the step of accessing, by the first computing device, a plurality of state data comprising information indicative of a state of a respective plurality of automated laboratory systems. Moreover, the method may comprise the step of selecting, by the first computing device, the second automated laboratory system out of the plurality of automated laboratory systems and assigning to the ALS2 the ALS2 set of actions. In particular, the aforementioned steps of: accessing the sample-processing data; obtaining the ALS1 state data; obtaining the ALS2 operation data; generating the ALS1 processing data; and initiating the generation of an ALS1 route plan may be carried out after the step of selecting the ALS2 and the step of assigning the ALS2 set of actions to the ALS2.

The step of selecting the ALS2 may comprise checking that the second ALS2 fulfils one or more requirements, the one or more requirements comprising, for instance, the requirement that the ALS2 is configured and available to carry out the ALS2 set of actions. Moreover, the selecting of the ALS2 may depend on the sample location data. For example if the sample is located at the ALS2, e.g. because an operator has loaded the sample in said ALS, ALS2 is selected, provided that the ALS2 is configured and available to carry out the ALS2 set of actions. This way, the processing time of the sample is reduced, as the sample is not moved to another ALS to carry out the ALS2 set of actions.

As mentioned, the ALS2 operation data comprise information indicative of an ALS2 set of actions of at least a first procedure, i.e. one or more actions comprised in one or more procedures In some examples, the ALS2 set of actions may be part of one or more procedures that are not included in the set of procedures of the sample-processing data. In other examples, the at least first procedure may belong to the set of procedures of the sample-processing data.

Exemplarily, the ALS2 set of actions may constitute one or more complete procedures. For instance, the ALS2 set of actions may constitute a first procedure that the ALS2 is configured to carry out, i.e. the first procedure may consist of the ALS2 set of actions.

The one or more actions of the ALS2 set have been either carried out or are scheduled to be carried out by the second ALS. In other words, if the ALS2 operation data are obtained at a timepoint t₀, the actions may have been performed at any timepoint preceding to or may be scheduled to be performed at any timepoint following to. Accordingly, the ALS2 set of actions may generally be a set of actions assigned to the ALS2 to be performed by it. The information indicative of the ALS2 set of actions may comprise indications of which actions were/will be performed, e.g. a list of actions that the ALS2 carried out or will carry out.

The method further comprises generating, by the first computing device, ALS1 processing data by using the sample-processing data, the ALS1 state data and the ALS2 operation data, wherein the ALS1 processing data comprise information indicative of an ALS1 set of actions of at least a second procedure of the set of procedures of the sample-processing data.

Accordingly, the step of generating ALS1 processing data may comprise assigning at least one procedure (the second procedure) of the set of procedures to the ALS1. Specifically, the ALS1 processing data may comprise information indicative of a selection (or subset) of the actions of the assigned procedures. Accordingly, the ALS1 processing data may comprise information assigning one or more actions to the ALS1, which will be carried out by the ALS1 at a timepoint lying in the future with respect to the timepoint at which the ALS1 processing data are generated.

Exemplarily, the ALS1 set of actions does not comprise at least one of the actions of the ALS2 set of actions. Said otherwise, the ALS2 set of actions may comprise at least one action that is not part of the ALS1 set of actions. In some examples, the ALS1 set of action and the ALS2 set of actions may be disjoint sets.

In particular, the first computing device may determine, based on the previously accessed/obtained data, which procedure(s) of the set of procedures shall be performed by the first ALS according to the capabilities of the ALS1 as specified by the ALS1 state data. At least the second procedure shall be performed by the ALS1.

Then, the first computing device may obtain a first set of actions, which consists of the actions comprised in each of said procedures (e.g. the second procedure), and select a subset of said actions. The criteria for the selection may be provided by the ALS1 state data and the ALS2 operation data. In particular, the first computing device obtains the ALS1 set of actions by excluding the actions of the first set of actions that, according to the ALS2 operation data, have been/will be performed by the ALS2. For instance, the second procedure may consist of the first set of actions.

Accordingly, the ALS1 set of actions may be a proper subset of the actions comprised in the procedures assigned to ALS1. In particular, the ALS1 set of actions, SA1, may be obtained by excluding from the first set of actions, SP, those actions that are present in the ALS2 set of actions, SA2. In mathematical terms, in this case SA1 is the difference between SP and SA2, i.e. SA1=SP\SA2.

For example, if the sample-processing data comprise information indicative of one or more procedures without indications about the actions, the first computing device may retrieve the one or more actions comprised in a procedure, for example by looking up an association between a procedure and its actions (e.g. stored in a table).

The information indicative of the ALS1 set of actions may be provided in a "positive" way, i.e. specifying which actions shall be performed, or in a "negative" way, i.e. specifying which actions comprised in the set of procedures assigned to ALS1 shall not be performed by the ALS1.

Based on the above, in a particular example, the first procedure may consist of the ALS2 set of actions, the second procedure may consist of a first set of actions and the ALS1 set of actions may consist of those actions of the first set of actions that are not in the ALS2 set of actions.

In a particular example, the step of generating ALS1 processing data may further comprise: obtaining information indicative of each action that is comprised in the at least second procedure and fulfils a set of selection requirements. In particular, the information indicative of said each action is comprised in the sample processing data, either directly or indirectly, as discussed above.

Exemplarily, the set of selection requirements comprises, e.g. consists of the requirement of not be comprised in the ALS2 set of actions. For instance, If the set of selection requirements comprises the requirement of not be comprised in the ALS2 set of actions, in order for an action to fulfil the set of selection requirements, the action shall not be comprised in the ALS2 set of actions.

In another example, other sets of actions may be defined, e.g. an ALS3 set of actions of actions performed by a third ALS, and the set of selection requirements may comprise the requirement of not being comprised in the ALS3 set of actions. More generally, a plurality of selection requirements may be defined and the first computing device may obtain information indicative of each action that is comprised in the at least second procedure and fulfils the plurality of selection requirements. For example, if the set of selection requirements consists of the requirement of not being comprised in the ALS2 set of actions and the requirement of not being comprised in the ALS3 set of actions, an action fulfils the set of selection requirements if it is comprised neither in the ALS2 set of actions nor in the ALS3 set of actions.

In particular, the step of generating the ALS1 processing data further comprises generating the ALS1 processing data by using the information indicative of each action that is comprised in the procedure and fulfils the set of selection requirements.

It should be noted that it is not requested that a check of whether the set of selection requirement is fulfilled be performed before obtaining the information. This may be the case in some examples. In other examples, information indicative of all actions in the procedure may be obtained and then only those actions fulfilling the set of selection requirements may be included in the ALS1 processing data.

Thus, exemplarily, the step of generating ALS1 processing data may further comprise, for each action that is comprised in the procedure:
- assessing whether said each action fulfils the set of selection requirements; and
- if said each action fulfils the set of selection requirements, obtaining information indicative of said each action.

The method further comprises initiating, by the first computing device, the generation of an ALS1 route plan for the first automated laboratory system, the ALS1 route plan being based on the ALS1 processing data. In other words, the first computing device causes the generation of the ALS1 route plan and it may do so by creating the plan itself or by prompting another device to do it.

In a particular example, initiating the generation of the ALS1 route plan for the first automated laboratory system may comprise, e.g. consists of, providing the ALS1 processing data to a second computing device, the second computing device being configured to generate the ALS1 route plan for the first automated laboratory system by using the ALS1 processing data. For instance, initiating the generation of the ALS1 route plan for the first automated laboratory system may further comprise instructing the second computing device to generate the ALS1 route plan.

In particular, the second computing device may generate the ALS1 route plan directly upon receipt of the ALS1 processing data. In other words, receiving the ALS1 processing data may trigger the generation of the route plan, without the need of specific instructions. Alternatively, the second computing device may generate the ALS1 route plan after receipt of the ALS1 processing data and further upon receiving instructions to generate the ALS1 route plan, e.g. from the first computing device. In other examples, the instructions to generate the ALS1 route plan may be received from the ALS2.

In another particular example, initiating the generation of the ALS1 route plan for the first automated laboratory system may comprise generating, by the first computing device, the ALS1 route plan for the first automated laboratory system by using the ALS1 processing data.

The first ALS is configured to process the biological sample according to the ALS1 route plan. Generally, a route plan of an ALS comprises information indicative of how to process the biological sample by that ALS. In other words, an ALS route plan comprises one or more processing steps, e.g. a chronological sequence thereof. In particular, the ALS1 route plan may comprise at least one processing step for performing the ALS1 set of actions.

Indeed, the ALS1 route plan may identify one or more ALS1 components (e.g. laboratory instruments) that are expected to carry out the ALS1 set of actions specified by the ALS1 processing data. In case of a plurality of components, the route plan may indicate a path through the plurality of components that the sample should follow. In other words, the route plan may specify an order according to which the sample should be handled by the different components of the ALS1, i.e. a temporal sequence of processing steps. Some processing steps may be carried out one after the other, other processing steps may be carried out with at least a partial time overlap, yet other processing steps may be carried out at any point in time and may, thus, not be included in the temporal sequence (while still being part of the route plan). Accordingly, the route plan may comprise an ordered list of processing steps that each component should carry out.

The computing device that generates the ALS1 route plan may associate one or more processing steps to each action indicated in the ALS1 processing data, i.e. assigned to the ALS1. For example, the computing device may identify a component (e.g. a centrifuge) capable of carrying out an action (e.g. centrifuging the sample) and may complement the action with specific parameters (e.g. speed, duration) contingent e.g. on the component. The parameters may be accessed from a data storage in which associations between the parameters and the ALS component are stored.

Additionally or alternatively, the parameters may be contingent on the sample type and/or on the procedure of which the action is part. Accordingly, the data storage may comprise different kinds of associations, e.g. including associations between parameters and procedures. Thus, in some examples, information linking an action of the ALS1 set of actions to the procedure (e.g. the second procedure) it belongs to may be needed when setting the parameters. This information may be part of the ALS1 processing data, i.e. the ALS1 set of actions may be associated to the procedure(s) it belongs to within the ALS1 processing data. In other examples, the route plan may be generated also based on other data containing such information, e.g. the sample-processing data.

The route plan may include processing steps involving transportation of the sample, such as from an input module to an instrument or from an instrument to an output module or between instruments. The computing device may access layout data representing the spatial arrangement of the ALS components in order to determine processing steps involving transportation of the sample. Accordingly, the route plan may be generated also based on layout data, in particular based on the distance or transportation time between various components within the ALS.

The execution of the computer-implemented method according to the present disclosure leads to the generation of a route plan, the ALS1 route plan, which prescribes how the biological sample shall be processed by the ALS1.

Since the ALS1 route plan is based on the ALS1 processing data which are generated using the sample-processing data, the ALS1 state data, and the ALS2 operation data, the ALS1 route plan allows for a more efficient and more accurate processing of the biological sample. In particular, the ALS1 route plan generated according to the described method has the effect of reducing sample contamination and/or consumption by avoiding the repetition of actions/processing steps.

Indeed, conventionally, each procedure is treated as an inseparable block which is assigned to a given ALS and the route plan is made considering only the given ALS and the procedure as a whole. Instead, the described method treats procedures at the granular level of actions. This allows for determining whether the same action(s) are comprised in more than one procedure and can, thus, be performed only once at one ALS instead of multiple times at different ALSs for each procedure. In particular, a given action may consume part of the sample and/or potentially contaminate or destroy the sample: by performing the given action only once, the quantity of used-up sample is minimized and the risk of contamination/destruction is decreased.

Furthermore, the granular approach allows for a distribution of different actions within the same procedure to different ALSs, wherein each action may be assigned to the ALS that can carry it out best, e.g. within the shortest time and/or with the optimal quality. In other words, the processing performance is considered at a multi-ALS level. Therefore, the quality of the sample processing and/or the processing time are improved by distributing actions and/or procedures across different ALSs.

The processing time of a biological sample is the amount of time elapsing between a timepoint in which the biological sample is ready to be processed (e.g. ready to be transported to a laboratory) and a timepoint in which the processing of the biological sample is completed.

In a particular example, obtaining the ALS2 operation data may comprise the step of accessing ALS2 state data, wherein the ALS2 state data comprise information indicative of a state of the second automated laboratory system, and the step of generating the ALS2 operation data by using the sample-processing data and the ALS2 state data. In particular, in this case, the ALS2 operation data comprise information indicative of an ALS2 set of actions that is scheduled to be carried out by the ALS2, i.e. not yet performed.

The ALS2 state data may comprise the same kind of information comprised in the ALS1 state data described herein. The information comprised in the former state data, however, refers to the ALS2 instead of the ALS1. In general, the information comprised in the ALS2 state data is different than the information comprised in the ALS1 state data, e.g. as, in general, the state, availability, suitability of ALS1 is different than the state, availability, suitability of ALS2, respectively. The first computing device may, thus, based on the state of the ALS2, in particular the suitability of the ALS2, determine which procedures of the sample-processing data should be assigned to the ALS2.

In one example, the first computing device may further initiate the generation of an ALS2 route plan for the ALS2, the ALS2 route plan being based on the ALS2 operation data, wherein the ALS2 route plan comprises at least one processing step for implementing the ALS2 set of actions.

Furthermore, the first computing device may determine which ALS should process the sample first. Indeed, in this particular example, the biological sample may have not yet been processed by either of the two ALSs when the method is executed. The method may comprise determining, by the first computing device, by using the ALS1 state data and the ALS2 state data (and optionally the sample-processing data), that the sample should be processed by the ALS2 first and by the ALS1 later. In other words, it may be determined that the sample is to be processed according to a temporal sequence in which the sample undergoes processing at the ALS2 and subsequently undergoes processing at the ALS1.

In a particular example, the method may further comprise the step of instructing the first automated laboratory system to process the biological sample using the ALS1 route plan. For example, the instructing may be performed by the computing device within the ALS1, which may be the first computing device or the second computing device.

Subsequently, the ALS1 may process the sample using the ALS1 route plan.

Additionally, the method may further comprise the step of accessing, by the first computing device, error data, wherein the error data comprise information indicative of a processing error of the second automated laboratory system in processing the biological sample; and the step of initiating, by the first computing device, the instructing of the second automated laboratory system to move the biological sample into an output module of the second automated laboratory system.

In particular, the step of initiating the instructing of the ALS2 to move the biological sample into the output module may be performed in response to accessing (e.g. receiving) the error data.

Generally, if a (second) step is performed after a (first) step (or, in particular, a computing device is configured to perform the second step after the first step), the second step occurs temporally after the first step and may or may not be triggered by the first step. In particular, considering that a step has a start, a finite duration and an end, a second step may be performed after the first step if at least the start of the second step is subsequent to the start of the first step. In some cases, the start of the second step may be subsequent to the end of the first step. If the first step is not carried out, the second step may be still carried out.

If a second step is performed in response to a first step, not only does the second step occur temporally after the first step, but performing the second step is conditional on performing the first step. In other words, the fact that the first step is carried out triggers the execution of the second step. If the first step is not carried out, the second step is also not carried out.

The processing error may be an event in the execution of a given processing step by the ALS2 or may be an event within the ALS2 that prevents a given processing step from being performed at all. In other words, the error may occur prior to a processing step that is supposed/planned to be performed by the ALS2 or the error may occur while the processing step is carried out, leading to the processing step being aborted.

The event may be a failure of (at least part of) the ALS2, such as a malfunctioning, or it may be an alert received by the computing device of the ALS2 that causes the ALS2 not to perform the processing step. For instance, the alert may indicate that a processing step may be performed, but only with a sub-optimal quality.

Initiating the instructing may be carried out by providing instruction data to an operator and the operator may in turn instruct, e.g. by using an input device of the second ALS, the second ALS to move the biological sample into the output module of the ALS2. Alternatively or in conjunction with the above, initiating the instructing may be carried out by instructing, by the first computing device, the second ALS to move the biological sample into an output module of the second automated laboratory system. For example, if a third computing device is comprised in the second ALS and controls the components of the second ALS, the first computing device may instruct the third computing device. The third computing device may, in turn, instruct the components of the second ALS to move the biological sample into an output module of the ALS2.

In this example, error data are used to prevent or address an incorrect processing of the biological sample. Furthermore, the biological sample is prepared to leave the ALS2 once the first computing device is made aware of the processing error. Accordingly, the processing of the sample is made more efficient.

In response to the processing error occurred in the ALS2, the first computing device may look for another ALS to perform the one or more processing steps that the ALS2 could not carry out. In some examples, the other ALS may be the ALS1. Accordingly, the aforementioned steps of: accessing the sample-processing data; obtaining the ALS1 state data; obtaining the ALS2 operation data; generating the ALS1 processing data; and initiating the generation of an ALS1 route plan may be carried out in response to the step of accessing error data.

In this case, the ALS1 backs up the ALS2 and, hence, allows for continuing the processing of the sample. The sample is processed according to the ALS1 route plan which is generated by taking into account the actions already taken by the ALS2 thereby preventing the ALS1 from performing twice time-consuming actions that may potentially lead to contamination or sample destruction.

In particular, the first procedure and the second procedure may coincide. The union of the ALS1 set of actions and the ALS2 set of actions may be the set of all actions comprised in the procedure.

In some examples, the method may further comprise the step of accessing, by the first computing device, a plurality of state data comprising information indicative of a state of a respective plurality of automated laboratory systems.

Exemplarily, the first computing device may be in communication with a plurality of ALSs and may access the state data of each of these ALSs. The first computing device may check from the state data which ALS may be suitable to perform the processing steps/actions that the ALS2 could not perform.

In some examples, the information indicative of which steps/actions could not be carried out by the ALS2 may be fully comprised in the error data. In other examples, the step of selecting may be carried out by using, in addition to the error data and the plurality of state data, the ALS2 operation data and/or the sample-processing data. In particular, the information in the error data may be combined with the information in the ALS2 operation data and/or the sample-processing data to determine which steps/actions were not carried out by the ALS2.

The method may further comprise the step of selecting, by the first computing device, the first automated laboratory system out of the plurality of automated laboratory systems by using at least the error data and the plurality of state data. As discussed above, for example, the aforementioned steps of: accessing the sample-processing data; obtaining the ALS1 state data; obtaining the ALS2 operation data; generating the ALS1 processing data; and initiating the generation of an ALS1 route plan, may be carried out after the step of accessing error data and in response to the step of selecting the first ALS.

Alternatively, the method may comprise the step of selecting, by the first computing device, a third automated laboratory system out of the plurality of automated laboratory systems by using at least the error data and the plurality of state data.

Accordingly, the first computing device may select the ALS that can perform the missed processing steps/actions, which is referred to as "third automated laboratory system" (or "ALS3" or "third ALS"). Said otherwise, the chosen ALS is the one whose state data indicate a suitability for carrying out the actions of the ALS2 set of actions that could not be carried out by the ALS2.The state data comprising information indicative of a state of the third automated laboratory system are referred to as "ALS3 state data". If more than one ALS is capable of carrying out the processing steps/actions, the selection may further be based on quality and/or speed of operation, as indicated by the state data, or on a location of the ALSs (e.g. the ALS closer to the ALS2 may be selected).

In this example, state data are used to set up a correct and swift continuation of the processing of the biological sample in case of errors. Accordingly, the processing of the sample is made more efficient and accurate.

Continuing with this particular example, the method may further comprise the step of generating, by the first computing device, ALS3 processing data by using at least the ALS3 state data and the error data; and the step of initiating, by the first computing device, the generation of an ALS3 route plan for the third automated laboratory system, the ALS3 route plan being based on the ALS3 processing data.

The ALS3 processing data may be generated in a similar manner as the ALS1 processing data described above and may comprise an ALS3 set of actions to be performed by the ALS3. In view of the discussion above, the sample is processed by the ALS3 after having been at the ALS2. Further, in particular, the sample may be processed by the ALS3 before being moved to the ALS1. The ALS3 may compensate for the error at the ALS2 and finish carrying out the ALS2 set of actions. In this way, if the first computing device had generated the ALS1 processing data based on the ALS2 set of actions scheduled to be carried out by the ALS2, the processing of the sample at the ALS1 can proceed as planned and it is not affected by the error occurred at the ALS2.

In particular, the ALS3 set of actions may be a proper subset of the ALS2 set of actions, wherein the actions that were already performed by the ALS2 are not included in the ALS2 set of actions. As discussed above, in addition to the error data, the information in the ALS2 operation data and/or sample-processing data may be used to determine which actions of the ALS2 set of actions were or were not carried out by the ALS2.

The ALS3 route plan may be generated in a similar manner as the ALS1 route plan described above and may comprise at least one processing step for implementing the ALS3 set of actions.

Generally, the transfer of the biological sample from one ALS to another ALS, e.g. from the ALS2 to the ALS1 or from the ALS2 to the ALS3, may be, in one example, performed automatically, e.g. by means of conveyors and/or robotic arms. In another example, a user may manually carry the sample from one ALS to the other ALS.

Once again, it is avoided that processing steps are unnecessarily repeated, thereby reducing the risk of contamination/destruction of the sample and minimising the quantity of used-up sample. Further, it is prevented that an error at one ALS affects another ALS. In particular, it is ensured that no action is mistakenly skipped. For example, if the ALS1 was not supposed to perform a given action, according to the ALS1 route plan, because the ALS2 was supposed to perform that given action, the ALS3 will now carry out the given action.

In the example above, the first computing device addresses the error at the ALS2 by involving the ALS3, so that nothing changes for the ALS1. Alternatively, the first computing device may update the ALS1 route plan to account for an unexpected occurrence at the ALS2, such as an error. In a particular example, after initiating the generation of the ALS1 route plan, the method may further comprise the steps of:
- obtaining, by the first computing device, second ALS2 operation data, wherein the second ALS2 operation data comprise information indicative of a second ALS2 set of actions that were not or will not be carried out on the biological sample by the second automated laboratory system, wherein the second ALS2 set of actions is a subset of the ALS2 set of actions;
- generating, by the first computing device, second ALS1 processing data by using the second ALS2 operation data and at least one of the ALS1 processing data and the sample-processing data, wherein the second ALS1 processing data comprise information indicative of a second ALS1 set of actions; and
- initiating, by the first computing device, the generation of a second ALS1 route plan for the first automated laboratory system, the second ALS1 route plan being based on the second ALS1 processing data, wherein the second ALS1 route plan comprises at least one processing step for implementing the second ALS1 set of actions.

The ALS2 operation data, the ALS2 set of actions, the ALS1 processing data, the ALS1 set of actions, the ALS1 route plan described heretofore may also be referred to as first ALS2 operation data, first ALS2 set of actions, first ALS1 processing data, first ALS1 set of actions, first ALS1 route plan, respectively.

This particular example may refer to a scenario in which, after the generation of the first ALS1 route plan has been initiated, but before the ALS1 starts processing the biological sample, an event occurs that modifies the set of actions that the ALS2 was scheduled to carry out according to the first ALS2 operation data.

In some examples, the event may comprise a change in the state of the ALS2. For instance, the event may comprise one or more of the following: a malfunctioning of one or more instruments of the ALS2, an unexpected change in the workload of the ALS2, a change in the expected quality of operation due e.g. to scarcity of a reagent, and so on.

The occurrence of the event at the ALS2 may prevent the ALS2 from performing one or more actions of the first ALS2 set of actions. Additionally or alternatively, the occurrence of the event at the ALS2 may prompt the first computing device to re-assign one or more actions of the first ALS2 set of actions, e.g. to meet time constraints or quality constraints. Exemplarily, the first computing device may monitor the ALS2 continuously or at given time intervals, since the state of the ALS may evolve with time, e.g. according to a data-push model and/or a data-pull model.

Accordingly, the second ALS2 operation data comprise information indicative of a second ALS2 set of actions that were not or will not be carried out on the biological sample by the second automated laboratory system, due to the event occurred at the ALS2. Since originally the first ALS2 set of actions was scheduled to be carried out by the ALS2, the second ALS2 set of actions may be a proper subset of the first ALS2 set of actions or may coincide with the first ALS2 set of actions, in case the ALS2 could not/will not perform any action.

In other examples, the event may comprise the assignment of additional procedure(s) to the ALS2 with respect to the procedure(s) that was/were originally assigned to it. In this case, the second ALS2 operation data comprise information indicative of a second ALS2 set of actions that has been or will be carried out on the biological sample by the second automated laboratory system and the second ALS2 set of actions is a superset of the first ALS2 set of actions.

Exemplarily, the second ALS2 operation data may be considered an updated version of the first ALS2 operation data, with the first ALS2 operation data being obtained at a first timepoint tₒ₁ and the second ALS2 operation data being obtained at a second timepoint tₒ₂ and with tₒ₂> tₒ₁. In some examples, the second ALS2 operation data may be obtained by modifying the first ALS2 operation data, e.g. by removing one or more actions from the first ALS2 set of actions. The removed actions may be those that were not/will not be carried out by the ALS2 or may be those that were/will be carried out by the ALS2 ("positive/negative" information as discussed above).

The second ALS1 processing data are generated by using the second ALS2 operation data and at least one of the first ALS1 processing data and the sample-processing data. For example, if the second ALS2 operation data comprise "positive" information about which actions were not/will not be carried out by the ALS2, e.g. a list of the second ALS2 set of actions, the first computing device may use the first ALS1 processing data and add the second ALS2 set of actions to the first ALS1 set of actions to obtain the second ALS1 set of actions. Accordingly, the second ALS1 processing data may be obtained by modifying the first ALS1 processing data.

In another example, the first computing device may use the second ALS2 operation data with the sample-processing data to generate the second ALS1 processing data "from scratch". In this case, the ALS1 state data may also be used. In other examples, both the first ALS1 processing data and the sample-processing data may be used, and optionally the ALS1 state data too. Exemplarily, in cases in which the (first) ALS1 state data are used, the first computing device may access second ALS1 state data obtained at a later timepoint and the second ALS1 state data may be used in place of the first ALS1 state data.

In some examples, the first ALS1 set of actions is a proper subset of the second ALS1 set of actions. Indeed, new actions are assigned to the ALS1 that will no longer be performed by the ALS2. In other examples, as discussed above, the ALS2 may perform more actions than initially planned, so that less actions are assigned to the ALS2 than initially planned. In this case, the second ALS1 set of actions is a proper subset of the first ALS1 set of actions.

The second ALS1 route plan may be generated similarly to the first ALS1 route plan as discussed above. Exemplarily, the second ALS1 route plan may be obtained by modifying the first ALS1 route plan.

In this particular example, a correct and efficient processing of the sample is achieved. Indeed, the first computing device can efficiently transfer procedures/actions from the ALS2 to the ALS1 if there any issues with the ALS2. Furthermore, it is ensured that no action is mistakenly skipped. For example, if the ALS1 was not supposed to perform a given action, according to the first ALS1 route plan, because the ALS2 was supposed to perform that given action, obtaining the updated second ALS2 operation data leads to a correction of the original ALS1 route plan resulting in the second ALS1 route plan, which comprises the given action.

According to a second aspect of the invention, a computer-implemented method is provided. The computer-implemented method comprises the steps of:
- accessing, by a second computing device, ALS1 processing data from a first computing device, wherein:
   - the ALS1 processing data comprise information indicative of an ALS1 set of actions of at least a second procedure of a set of procedures to be carried out on a biological sample, wherein each procedure comprises one or more actions, and
   - the first computing device is configured to generate the ALS1 processing data by using sample-processing data, ALS1 state data and ALS2 operation data; and
      - generating, by the second computing device, an ALS1 route plan for a first automated laboratory system, the ALS1 route plan being based on the ALS1 processing data;
      wherein:
- the sample-processing data comprise information indicative of the set of procedures,
- the ALS1 state data comprise information indicative of a state of a first automated laboratory system - ALS1, and
- the ALS2 operation data comprise information indicative of an ALS2 set of actions of at least a first procedure, wherein the ALS2 set of actions either has been carried out or is scheduled to be carried out by a second automated laboratory system - ALS2 - on the biological sample.

The description of the first aspect applies *mutatis mutandis* to the second aspect of the invention.

As already explained in connection with the first aspect, "accessing ALS1 processing data" may comprise "receiving the ALS1 processing data", e.g. in response to a request for the ALS1 processing data sent by the second computing device to the first computing device. Alternatively, the ALS1 processing data may be sent by the first computing device without a request/prompt of the second computing device. In another example, "accessing ALS1 processing data" may comprise "retrieving the ALS1 processing data" by the second computing device from the first computing device, e.g. by accessing a memory of the first computing device or an external storage. For instance, the first computing device may upload the ALS1 processing data to an external storage, e.g. to a cloud storage, and "accessing (retrieving) ALS1 processing data" may comprise "downloading the ALS1 processing data" from the external storage by the second computing device.

The second computing device is configured to generate the ALS1 route plan using the ALS1 processing data. For example, the second computing device may identify the component(s) in the ALS1 that can perform the ALS1 set of actions specified by the ALS1 processing data.

According to a further aspect of the invention, a data processing system is provided. The data processing system comprise a processor configured to carry out the method described herein.

In particular, the first computing device may be a data processing system comprising a processor configured to carry out the method according to the first aspect of the invention.

The second computing device may be a data processing system comprising a processor configured to carry out the method according to the second aspect of the invention.

In one example, the first ALS may comprise the first computing device and the second ALS may comprise the second computing device. Alternatively, the first computing device may be comprised in the second ALS and the second computing device may be comprised in the first ALS.

In another example, the first computing device may be not part of any ALS, the second computing device may be comprised in the first ALS and the third computing device may be comprised in the second ALS. The second computing device may be in data communication with the first computing device and may be configured to instruct the first ALS to process the biological sample according to the ALS1 route plan.

According to yet a further aspect of the invention, an automated laboratory system comprising the data processing system is provided.

If the second ALS comprises the second computing device, the second ALS may be in particular an automated laboratory system comprising a data processing system configured to carry out the method according to the second aspect of the invention.

According to another aspect of the invention, a computer program product is provided. The computer program product comprises instructions which, when the program is executed by a computer, cause the computer to carry out the method described herein.

The first computing device may comprise a computer program product comprising instructions which, when the program is executed by the first computing device, cause the first computing device to carry out the method according to the first aspect of the invention. The second computing device may comprise a computer program product comprising instructions which, when the program is executed by the second computing device, cause the second computing device to carry out the method according to the second aspect of the invention.

According to yet another aspect of the invention, a computer-readable medium is provided. The computer-readable medium comprises instructions which, when executed by a computer, cause the computer to carry out the methods described herein.

### Brief Description of the Drawings

Details of exemplary embodiments are set forth below with reference to the exemplary drawings. Other features will be apparent from the description, the drawings, and from the claims. It should be understood, however, that even though embodiments are separately described, single features of different embodiments may be combined to further embodiments.
Figure 1 shows a schematic representation of an exemplary first computing device, of an exemplary second computing device and of an exemplary third computing device.
Figure 2 shows a first swim lane diagram including exemplary steps performed by the first computing device, the second computing device and the third computing device.
Figures 3a and 3b show a second swim lane diagram including exemplary steps performed by the first computing device, the second computing device and the third computing device.
Figure 4 shows a third swim lane diagram including exemplary steps performed by the first computing device, the second computing device and the third computing device.
Figure 5 shows a fourth swim lane diagram including exemplary steps performed by the first computing device, the second computing device and the third computing device.

### Detailed Description

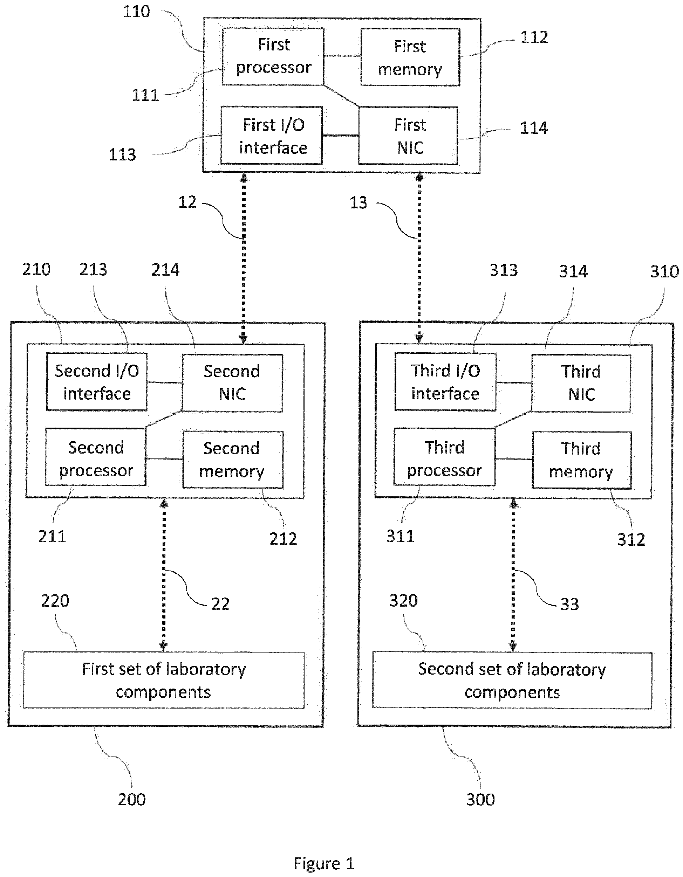

**Figure 1** shows a schematic representation of an exemplary first computing device, of an exemplary second computing device and of an exemplary third computing device. Each of the first, second and third computing device may comprise a plurality of processors and/or a plurality of memories, possibly spatially separated from one another and/or not included in the same casing. In particular at least some of those devices may be in the cloud, i.e. hosted in a cluster of servers in data communication with one another, or may constitute a computer network.

Exemplarily, the first computing device 110 comprises a first processor 111 (e.g. a CPU, a GPU, or the like), and a first memory 112. The first processor 111 is configured to carry out the method according to the first aspect described above. The first memory 112 may comprise a primary memory and a secondary memory (not shown). In particular, the secondary memory stores a computer program comprising instructions which, when executed by the processor 111, cause the first computing device 110 to carry out the method according to the first aspect described above. The first computing device 110 may also comprise a first input output (I/O) interface 113 for communicating with input/output units, such as a screen, a keyboard, a touch screen, a printer, or the like.

The second computing device 210 comprises a second processor 211 (e.g. a CPU, a GPU, or the like), and a second memory 212. The second processor 211 is configured to carry out the method according to the second aspect described above. The second memory 212 may comprise a primary memory and a secondary memory (not shown). In particular, the secondary memory stores a computer program comprising instructions which, when executed by the processor 211, cause the second computing device 210 to carry out the method according to the second aspect described above. The second computing device 210 comprises a second input output (I/O) interface 213 for communicating with a input/output unit, such as a screen, a keyboard, a touch screen, a printer, or the like.

The second computing device 210 is comprised in a first ALS 200 that comprises a first set of laboratory components 220. The laboratory components 220 comprise one or more of analytic instruments, pre-analytic instruments, post-analytic instruments, input/output modules, transportation components. In particular, the second computing device 210 is configured to control the laboratory components of the ALS1 200. The two-way communication between the second computing device 210 and the laboratory components 220 is schematically represented in Figure 1 by the double-headed dashed line 22.

The third computing device 310 comprises a third processor 311 (e.g. a CPU, a GPU, or the like), and a third memory 312. The third memory 312 may comprise a primary memory and a secondary memory (not shown). The third computing device 310 comprises a third input output (I/O) interface 313 for communicating with an input/output unit, such as a screen, a keyboard, a touch screen, a printer, or the like.

The third computing device 310 is comprised in a second ALS 300 that comprises a second set of laboratory components 320. The laboratory components 320 comprise one or more of analytic instruments, pre-analytic instruments, post-analytic instruments, input/output modules, transportation components. In particular, the third computing device 310 is configured to control the laboratory components of the ALS2 300. The two-way communication between the third computing device 310 and the laboratory components 320 is schematically represented in Figure 1 by the double-headed dashed line 33.

Accordingly, the second and the third computing devices 210, 310 are configured to generate the respective route plans and are configurated to implement the respective route plans.

The first computing device 110, the second computing device 210 and the third computing device comprise a first Network Interface Controller (NIC) 114, a second NIC 214 and a third NIC 314, respectively, configured to connect said devices with one or more networks (e.g. an intranet, the internet, a cellular network, or the like). In other examples, each computing device may comprise a plurality of NICs. Further, each NIC may be virtual. The first computing device 110 and the second computing device 210 may exchange data with one another via the first NIC 114 and the second NIC 214 by using a protocol suite such as TCP or IP, said protocol being schematically represented in Figure 1 by the double-headed dashed line 12. The first computing device 110 and the third computing device 310 may exchange data with one another via the first NIC 114 and the third NIC 314 by using a protocol suite such as TCP or IP, said protocol being schematically represented in Figure 1 by the double-headed dashed line 13.

In the following description, unless specified otherwise, the roles of the first ALS 200 (and, thus, of the second computing device 210) and of the second ALS 300 (and, thus, of the third computing device 310) may be interchanged.

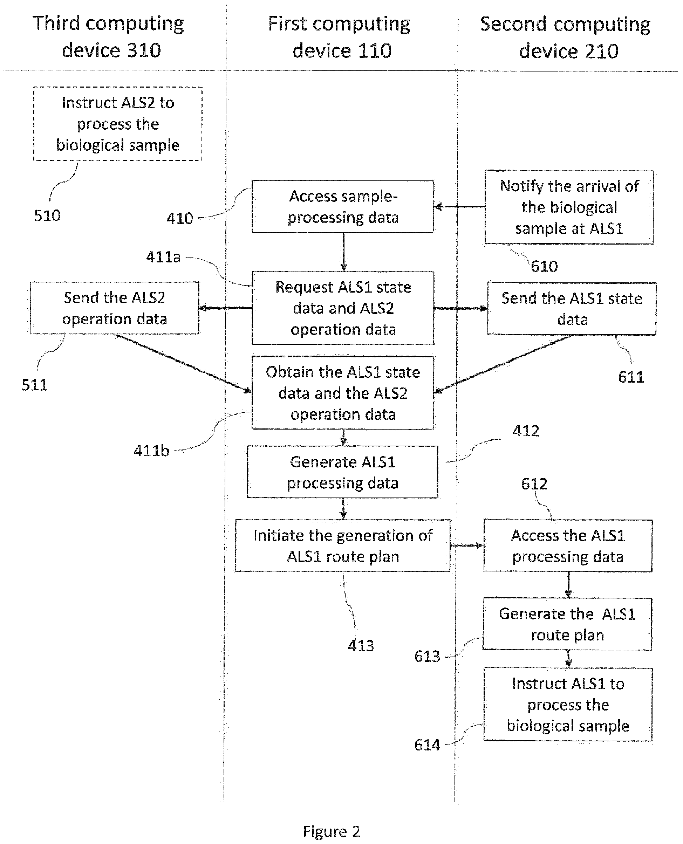

**Figure 2** shows a first swim lane diagram including exemplary steps performed by the first computing device, the second computing device and the third computing device.

At 410 the first computing device 110 accesses sample-processing data, wherein the sample-processing data comprise information indicative of a set of procedures to be carried out on a biological sample. For example, the step of accessing the sample-processing data may be performed in response to a notification that the sample has arrived at the ALS1 issued by the second computing device 210 (step 610). The sample may have been transported to the ALS1 from the ALS2 after having been processed at the ALS2, in particular based on instructions to process the sample, said instruction being provided by the third computing device 310 (step 510).

Exemplarily, at 411a the first computing device 110 may request the ALS1 state data to the second computing device 210 and request the ALS2 operation data to the third computing device 310. The second computing device 210 may send the ALS1 state data at 611 and the third computing device 310 may send the ALS2 operation data at 511, so that the first computing device 110 obtains the ALS1 state data and ALS2 operation data in that it receives them. Thus, the first computing device 110 obtains at 411b ALS1 state data comprising information indicative of a state of the ALS1 and ALS2 operation data comprising information indicative of an ALS2 set of actions, wherein the ALS2 set of actions was carried out by the ALS2 when processing the sample.

At 412, by using the sample-processing data, the ALS1 state data and the ALS2 operation data, the first computing device 110 generates ALS1 processing data, wherein the ALS1 processing data comprise information indicative of an ALS1 set of actions. In other words, the ALS1 processing data comprises information about which actions should be performed by the ALS1 when processing the sample.

At 413 the first computing device 110 initiates the generation of an ALS1 route plan, said generation being based on the ALS1 processing data. For example, at 612, the second computing device 210 may access (e.g. receive) the ALS1 processing data from the first computing device 110 and then, at 613, generate the ASL1 route plan, wherein the ALS1 route plan comprises at least one processing step for implementing the ALS1 set of actions. Finally, at 614, the second computing device 210 may instruct the ALS1 to process the biological sample according to the ALS1 route plan.

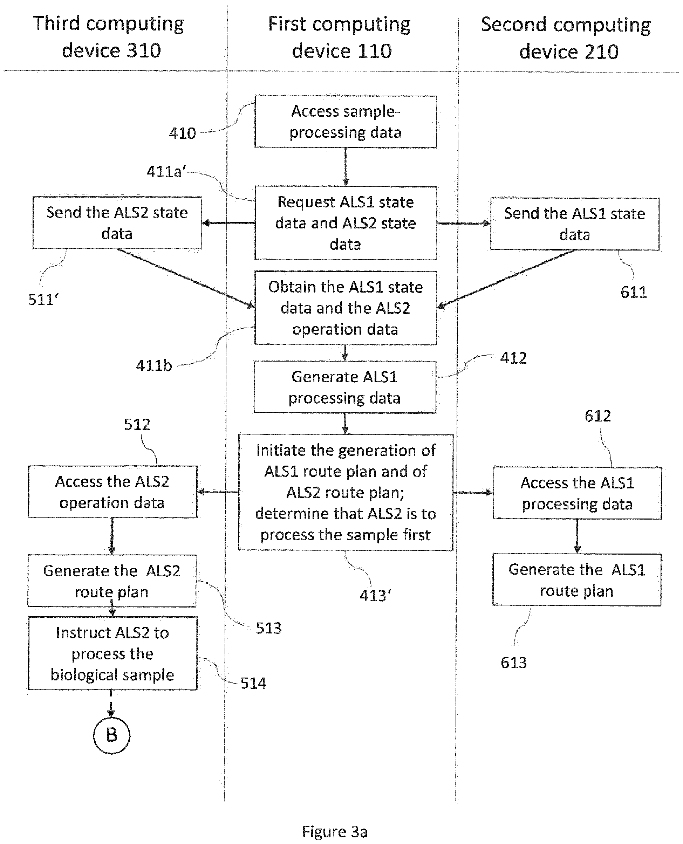

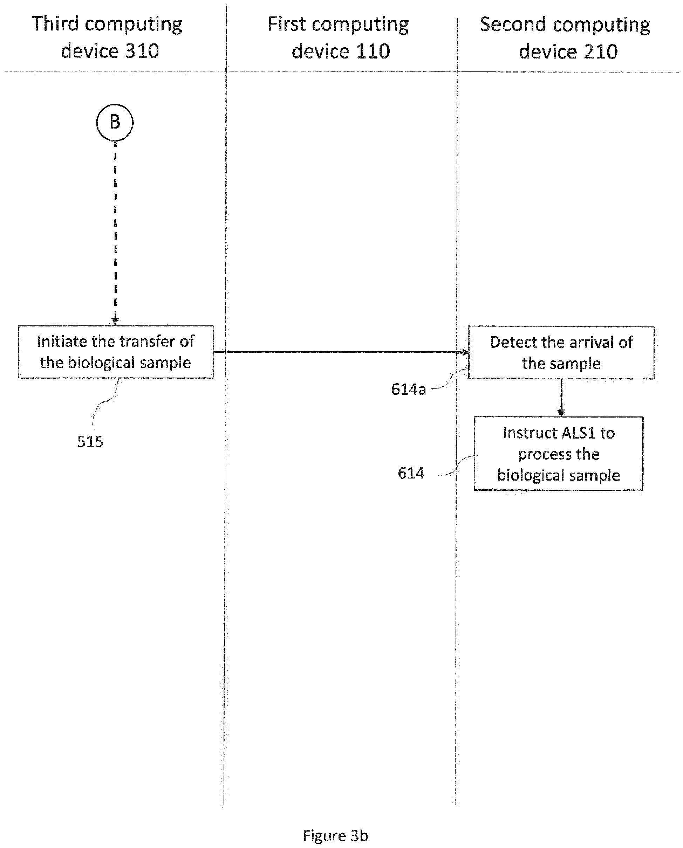

**Figures 3a** **and** **3b** show a second swim lane diagram including exemplary steps performed by the first computing device, the second computing device and the third computing device.

At 410, the first computing device 110 accesses sample-processing data, wherein the sample-processing data comprise information indicative of a set of procedures to be carried out on a biological sample. Then at 411a' the first computing device 110 may request ALS1 state data and ALS2 state data, wherein the ALS2 state comprise information indicative of a state of the ALS2. The second computing device 210 may send the ALS1 state data at 611 and the third computing device 310 may send the ALS2 state data at 511'.

Thus, the first computing device 110 obtains at 411b ALS1 state data comprising information indicative of a state of the ALS1 and ALS2 operation data comprising information indicative of an ALS2 set of actions. In particular, the first computing device 110 may obtain the ALS1 state data by receiving them from the second computing device 210 and may obtain the ALS2 operation data by using the received ALS2 state data and the sample-processing data to generate the ALS2 operation data. In this case, unlike in Figure 2, the ALS2 set of actions is scheduled to be carried out by the ALS2.

At 412, by using the sample-processing data, the ALS1 state data and the ALS2 operation data, the first computing device 110 generates ALS1 processing data.

At 413', the first computing device 110 initiates the generation of an ALS1 route plan, the generation being based on the ALS1 processing data. Furthermore, the first computing device 110 may initiate the generation of an ALS2 route plan, the generation being based on the ALS2 operation data. In this context, the first computing device 110 may also determine which ALS should process the sample first and may e.g. choose the ALS2 as first. This determination may be based on various factors, e.g. the type of actions that each ALS will carry (such as if one set of actions has a higher risk of contaminating/destructing the sample) and/or the location of the sample, as specified for example in the sample-processing data. For instance, the sample may already be at the ALS2 or, otherwise, subsequent to the determination that the ALS2 shall process the sample first, the first computing device 110 may trigger the arrival of the sample at the ALS2, e.g. by displaying instructions to a user.

Exemplarily, at 512 the third computing device 310 may access the ALS2 operation data and at 513 it may generate the ALS2 route plan being based on the ALS2 operation data. Afterwards, at 514, the third computing device 310 may instruct the ALS2 to process the sample according to the ALS2 route plan and finally, at 515, it may initiate the transfer of the sample to the ALS1. For instance, the ALS2 may bring the sample to an output module and transfer it from there to a conveyor configured to carry the sample to the ALS1.

At 614a the second computing device 210 may detect the arrival of the sample. While the sample was at the ALS2, the second computing device 210 may have accessed the ALS1 processing data at 612 and generated the ALS1 route plan at 613. In other examples, these two steps (or only step 613) may be carried out after detection of the arrival of the sample. Once the sample is at the ALS1 and the ALS1 route plan has been generated, at 614 the second computing device 210 instructs the ALS1 to process the sample according to the ALS1 route plan.

Exemplarily, in both scenarios of Figures 2 and 3, the ALS2 set of actions may be the complete set of actions needed to carry out a clinical test A and the clinical test A may include the action of centrifuging the sample. The ALS1 set of actions may be a proper subset of the actions needed to carry out a clinical test B, wherein the test B would also require a centrifuging action. However, since this action is already performed by the ALS2, the ALS1 set of actions is given by the complete set of actions needed to carry out the clinical test Y minus the centrifuging action. Centrifuging a sample is a mechanical action that entails a risk of destroying the sample: by centrifuging the sample once instead of twice, the risk is reduced by half. Further, avoiding the repetition of the centrifuging action makes the whole process more efficient time-wise.

In another example, the common action between test A and test B may be an invasive action that involves a risk of contamination of the sample, e.g. aliquoting for long-term storage. By performing the action only once, the overall risk of contamination is reduced, thereby improving the quality of the processing.

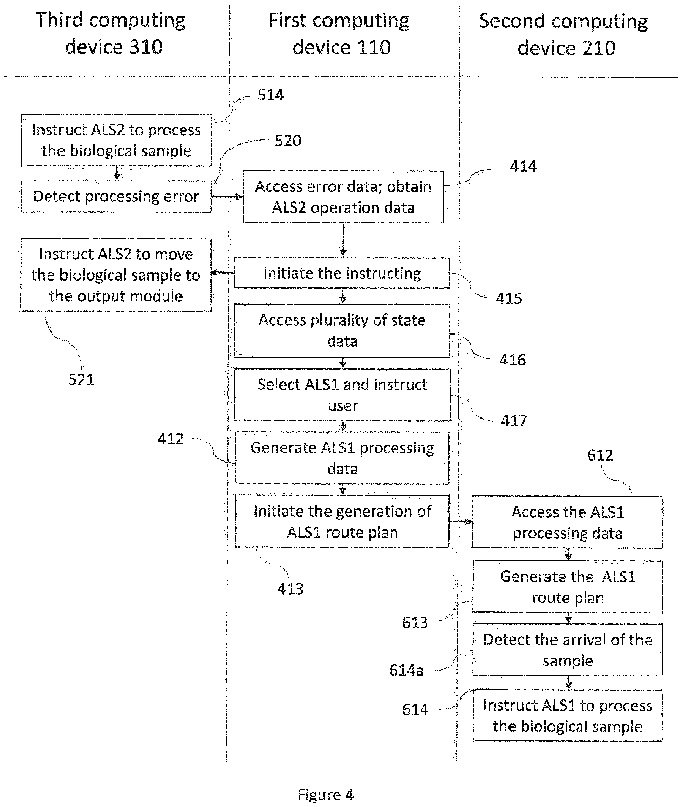

In some examples, further method steps may be performed by the computing devices. **Figure 4** shows a third swim lane diagram including exemplary steps performed by the first computing device, the second computing device and the third computing device.

At 514, the third computing device 310 may instruct the ALS2 to process the sample according to an ALS2 route plan. In particular, the ALS2 route plan may be generated by the third computing device 310. For example, the first computing device 110 may instruct the third computing device 310 to generate the ALS2 route plan based on an ALS2 set of actions.

While performing the ALS2 set of actions according to the ALS2 route plan, a processing error may occur at the ALS2, which may be detected by the third computing device 310 at 520. For example, an instrument of the ALS2 may break down while the sample is being processed by the ALS2, so that the processing step that was supposed to be carried out by that instrument can no longer be performed by that instrument and no other instrument on the ALS2 may be configured to perform that processing step.

The first computing device 110 may access (e.g. receive) error data comprising information indicative of the processing error at 414. In this example, the error data comprise ALS2 operation data. Hence, in this embodiment, the step of obtaining ALS2 operation data is carried out by accessing the error data.

For example, after accessing the error data, the first computing device 110 may at 415 communicate with the third computing device 310 and cause the third computing device 310 to instruct the ALS2 to move the sample to an output module of the ALS2 at 521. Alternatively, the third computing device 310 may on its own, after detecting the processing error, instruct the ALS2 to move the sample to an output module of the ALS2, e.g. to a region of the output module dedicated to transfer to the ALS1.

At 416 the first computing device 110 may access a plurality of state data comprising information indicative of a state of a respective plurality of ALSs, including ALS1 state data. At 417, by using the error data and the plurality of state data, the first computing device 110 may select the ALS1 out of the plurality of automated laboratory systems.

In particular, the ALS1 may be selected to compensate for the processing error occurred at the ASL2, namely to carry out the actions/processing steps that the ALS2 was supposed to perform but finally could not because of the processing error. The first computing device 110 determines, based on the ALS1 state data, that the ALS1 can carry out such actions/processing steps. The first computing device 110 may accordingly instruct a user, e.g. by displaying the instructions, to take the sample from the output module of the ALS2 and transport it to the ALS1.

At 412, by using the ALS1 state data and the error data (e.g. the ALS2 operation data comprised therein), the first computing device 110 may generate ALS1 processing data comprising information indicative of an ALS1 set of actions. At 413, the first computing device 110 may initiate the generation of an AL12 route plan, the route plan being based on the ALS1 processing data.

Accordingly, in one example, the second computing device 210 may access the ALS1 processing data at 612 and generate the AL12 route plan. Once the sample is at the AL12 (e.g. the second computing device 210 may detect the arrival of the sample at 614a after having generated the ALS1 route plan or, in other examples, before) and the ALS1 route plan has been generated, the second computing device 210 may instruct at 614 the ALS1 to process the biological sample according to the ALS1 route plan.

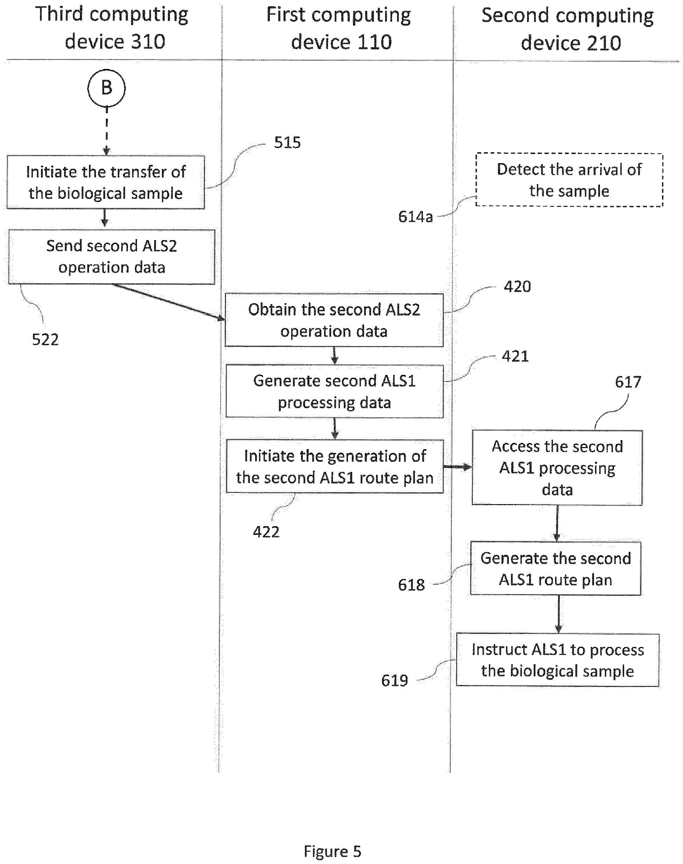

**Figure 5** shows a fourth swim lane diagram including exemplary additional steps performed by the first computing device, the second computing device and the third computing device. In a particular example, the steps shown in Figure 5 may be performed after the step 514 (or, more generally, after the ALS2 has processed the sample), as indicated by the letter "B" shown in Figures 3a and 5.

Exemplarily, when processing the sample, the ALS2 may have not carried out all actions that it was assigned to according to the (first) ALS2 operation data obtained at 411b, e.g. because of a malfunction. The second ALS2 operation data comprise information indicative of a second ALS2 set of actions that were not carried out on the biological sample by the ALS2, wherein the second ALS2 set of actions is a subset of the (first) ALS2 set of actions of the first ALS2 operation data.

At 515, the third computing device 310 may initiate the transfer of the sample to the ALS1. At 614a the second computing device 210 may detect the arrival of the sample. Step 614a may take place before steps 617 and 618 discussed below. In other examples, these two steps (or only step 618) may be carried out after detection of the arrival of the sample (step 614a). Similarly, step 515 may take place after step 522. At 522, the second computing device 210 sends the second ALS2 operation data to the first computing device 110.

The first computing device 110 may obtain the second ALS2 operation data at 420 and may at 421 generate second ALS1 processing data by using the second ALS2 operation data, the ALS1 processing data generated in step 412 (hereinafter: "first ALS1 processing data") and the sample-processing data accessed in step 410. The second ALS1 processing data comprise information indicative of a second ALS1 set of actions which is a superset of the ALS1 set of actions of the first ALS1 processing data (hereinafter: "first ALS1 set of actions"), since it comprises additional actions that were initially assigned to the ALS2 but will now have to be carried out by the ALS1.

Then, at 422, the first computing device 110 may initiate the generation of a second ALS1 route plan, said generation being based on the second ALS1 processing data. Accordingly, the second computing device 210 may access the second ALS1 processing data at 617 and generate the second ALS1 route plan at 618. The second ALS1 route plan may in particular replace the first ALS1 route plan generated at 613, if this step had already been performed. Finally, at 619, the second computing device 210 may instruct the ALS1 to process the sample according to the second ALS1 route plan.

The exemplary operations of the computing devices as illustrated in Figures 2 to 5 provide an optimal processing of a biological sample in terms of quality and efficiency. In particular, sample contamination and/or consumption is reduced by avoiding the repetition of actions/processing steps.

## Claims

1. Computer-implemented method comprising:
- accessing, by a first computing device, sample-processing data, wherein the sample-processing data comprise information indicative of a set of procedures to be carried out on a biological sample, wherein each procedure comprises one or more actions;
- obtaining, by the first computing device, ALS1 state data, wherein the ALS1 state data comprise information indicative of a state of a first automated laboratory system - ALS1;
- obtaining, by the first computing device, ALS2 operation data, wherein the ALS2 operation data comprise information indicative of an ALS2 set of actions of at least a first procedure, wherein the ALS2 set of actions either has been carried out or is scheduled to be carried out by a second automated laboratory system - ALS2 - on the biological sample;
- generating, by the first computing device, ALS1 processing data by using the sample-processing data, the ALS1 state data and the ALS2 operation data, wherein the ALS1 processing data comprise information indicative of an ALS1 set of actions of at least a second procedure of the set of procedures; and
- initiating, by the first computing device, the generation of an ALS1 route plan for the first automated laboratory system, the ALS1 route plan being based on the ALS1 processing data.

2. Method according to claim 1, wherein initiating the generation of the ALS1 route plan for the first automated laboratory system comprises generating, by the first computing device, the ALS1 route plan for the first automated laboratory system by using the ALS1 processing data.

3. Method according to any one of the preceding claims, wherein generating the ALS1 processing data further comprises:
obtaining information indicative of each action that is comprised in the at least second procedure and fulfils a set of selection requirements, wherein the set of selection requirements comprises the requirement of not being comprised in the ALS2 set of actions.

4. Method according to any one of the preceding claims, wherein obtaining the ALS2 operation data comprises:
accessing ALS2 state data, wherein the ALS2 state data comprise information indicative of a state of the second automated laboratory system;
generating the ALS2 operation data by using the sample-processing data and the ALS2 state data.

5. Method according to any one of the preceding claims, further comprising:
instructing the first automated laboratory system to process the biological sample using the ALS1 route plan.

6. Method according to any one of the preceding claims, further comprising:
accessing, by the first computing device, error data, wherein the error data comprise information indicative of a processing error of the second automated laboratory system in processing the biological sample;
initiating, by the first computing device, the instructing of the second automated laboratory system to move the biological sample into an output module of the second automated laboratory system.

7. Method according to the preceding claim, further comprising:
accessing, by the first computing device, a plurality of state data comprising information indicative of a state of a respective plurality of automated laboratory systems;
selecting, by the first computing device, a third automated laboratory system - ALS3 - out of the plurality of automated laboratory systems by using the error data and the plurality of state data, which include ALS3 state data comprise information indicative of a state of the third automated laboratory system.

8. Method according to the preceding claim, further comprising:
generating, by the first computing device, ALS3 processing data by using the ALS3 state data and the error data, wherein the ALS3 processing data comprise information indicative of an ALS3 set of actions;
initiating, by the first computing device, the generation of an ALS3 route plan for the third automated laboratory system, the ALS3 route plan being based on the ALS3 processing data.

9. Method according to any one of claims 1 to 5, further comprising, after initiating the generation of the ALS1 route plan:
obtaining, by the first computing device, second ALS2 operation data, wherein the second ALS2 operation data comprise information indicative of a second ALS2 set of actions;
generating, by the first computing device, second ALS1 processing data by using the second ALS2 operation data and at least one of the ALS1 processing data and the sample-processing data, wherein the second ALS1 processing data comprise information indicative of a second ALS1 set of actions;
initiating, by the first computing device, the generation of a second ALS1 route plan for the first automated laboratory system, the second ALS1 route plan being based on the second ALS1 processing data.

10. Method according to the preceding claim, wherein:
the second ALS2 set of actions is a subset of the ALS2 set of actions; or
the second ALS2 set of actions is a superset of the ALS2 set of actions.

11. Computer-implemented method comprising:
- accessing, by a second computing device, ALS1 processing data from a first computing device, wherein:
• the ALS1 processing data comprise information indicative of an ALS1 set of actions of at least a second procedure of a set of procedures to be carried out on a biological sample, wherein each procedure comprises one or more actions, and
• the first computing device is configured to generate the ALS1 processing data by using sample-processing data, ALS1 state data and ALS2 operation data; and
- generating, by the second computing device, an ALS1 route plan for a first automated laboratory system, the ALS1 route plan being based on the ALS1 processing data;
wherein:
- the sample-processing data comprise information indicative of the set of procedures,
- the ALS1 state data comprise information indicative of a state of a first automated laboratory system - ALS1, and
- the ALS2 operation data comprise information indicative of an ALS2 set of actions of at least a first procedure, wherein the ALS2 set of actions either has been carried out or is scheduled to be carried out by a second automated laboratory system - ALS2 - on the biological sample.

12. A data processing system comprising a processor configured to carry out the method according to any one of the preceding claims.

13. An automated laboratory system comprising the data processing system according to the preceding claim.

14. A computer program product comprising instructions which, when the program is executed by a computer, cause the computer to carry out the method according to any one of claims 1 to 11.

15. A computer-readable medium comprising instructions which, when executed by a computer, cause the computer to carry out the method according to any one of claims 1 to 11.

## Amended claims

### Amended claims in accordance with Rule 137(2) EPC.

1. Computer-implemented method comprising:
- accessing (410), by a first computing device (110), sample-processing data, wherein the sample-processing data comprise information indicative of a set of procedures to be carried out on a biological sample, wherein each procedure comprises one or more actions;
- obtaining (411b), by the first computing device (110), ALS1 state data, wherein the ALS1 state data comprise information indicative of a state of a first automated laboratory system (200) - ALS1;
- obtaining (411b), by the first computing device (110), ALS2 operation data, wherein the ALS2 operation data comprise information indicative of an ALS2 set of actions of at least a first procedure, wherein the ALS2 set of actions either has been carried out or is scheduled to be carried out by a second automated laboratory system (300) - ALS2 - on the biological sample;
the method **characterised by** further comprising:
- generating (412), by the first computing device (110), ALS1 processing data by using the sample-processing data, the ALS1 state data and the ALS2 operation data, wherein the ALS1 processing data comprise information indicative of an ALS1 set of actions of at least a second procedure of the set of procedures; and
- initiating (413), by the first computing device (110), the generation of an ALS1 route plan for the first automated laboratory system (200), the ALS1 route plan being based on the ALS1 processing data;
wherein generating the ALS1 processing data further comprises: obtaining information indicative of each action that is comprised in the at least second procedure and fulfils a set of selection requirements, wherein the set of selection requirements comprises the requirement of not being comprised in the ALS2 set of actions.

2. Method according to claim 1, wherein initiating (413) the generation of the ALS1 route plan for the first automated laboratory system (200) comprises generating, by the first computing device (110), the ALS1 route plan for the first automated laboratory system (200) by using the ALS1 processing data.

3. Method according to any one of the preceding claims, wherein obtaining (411b) the ALS2 operation data comprises:
accessing (411a', 511') ALS2 state data, wherein the ALS2 state data comprise information indicative of a state of the second automated laboratory system;
generating the ALS2 operation data by using the sample-processing data and the ALS2 state data.

4. Method according to any one of the preceding claims, further comprising:
Instructing (614) the first automated laboratory system (200) to process the biological sample using the ALS1 route plan.

5. Method according to any one of the preceding claims, further comprising:
accessing (414), by the first computing device (110), error data, wherein the error data comprise information indicative of a processing error of the second automated laboratory system (300) in processing the biological sample;
initiating (415), by the first computing device (110), the instructing of the second automated laboratory system (300) to move the biological sample into an output module of the second automated laboratory system (300).

6. Method according to the preceding claim, further comprising:
accessing (416), by the first computing device (110), a plurality of state data comprising information indicative of a state of a respective plurality of automated laboratory systems;
selecting, by the first computing device (110), a third automated laboratory system - ALS3 - out of the plurality of automated laboratory systems by using the error data and the plurality of state data, which include ALS3 state data comprise information indicative of a state of the third automated laboratory system.

7. Method according to the preceding claim, further comprising:
generating, by the first computing device (110), ALS3 processing data by using the ALS3 state data and the error data, wherein the ALS3 processing data comprise information indicative of an ALS3 set of actions;
initiating, by the first computing device (110), the generation of an ALS3 route plan for the third automated laboratory system, the ALS3 route plan being based on the ALS3 processing data.

8. Method according to any one of claims 1 to 4, further comprising, after initiating the generation of the ALS1 route plan:
obtaining (420), by the first computing device (110), second ALS2 operation data, wherein the second ALS2 operation data comprise information indicative of a second ALS2 set of actions;
generating (421), by the first computing device (110), second ALS1 processing data by using the second ALS2 operation data and at least one of the ALS1 processing data and the sample-processing data, wherein the second ALS1 processing data comprise information indicative of a second ALS1 set of actions;
initiating (422), by the first computing device (110), the generation of a second ALS1 route plan for the first automated laboratory system, the second ALS1 route plan being based on the second ALS1 processing data.

9. Method according to the preceding claim, wherein:
the second ALS2 set of actions is a subset of the ALS2 set of actions; or
the second ALS2 set of actions is a superset of the ALS2 set of actions.

10. Computer-implemented method comprising:
- accessing (612), by a second computing device (210), ALS1 processing data from a first computing device (110), wherein:
• the ALS1 processing data comprise information indicative of an ALS1 set of actions of at least a second procedure of a set of procedures to be carried out on a biological sample, wherein each procedure comprises one or more actions, and
• the first computing device (110) is configured to generate the ALS1 processing data by using sample-processing data, ALS1 state data and ALS2 operation data; and
- generating (613), by the second computing device (210), an ALS1 route plan for a first automated laboratory system (200), the ALS1 route plan being based on the ALS1 processing data;
wherein:
- the sample-processing data comprise information indicative of the set of procedures,
- the ALS1 state data comprise information indicative of a state of the first automated laboratory system (200) - ALS1,
- the ALS2 operation data comprise information indicative of an ALS2 set of actions of at least a first procedure, wherein the ALS2 set of actions either has been carried out or is scheduled to be carried out by a second automated laboratory system (300) - ALS2 - on the biological sample,
- the first computing device (110) is configured to generate the ALS1 processing data by obtaining information indicative of each action that is comprised in the at least second procedure and fulfils a set of selection requirements, wherein the set of selection requirements comprises the requirement of not being comprised in the ALS2 set of actions.

11. A data processing system comprising a processor configured to carry out the method according to any one of the preceding claims.

12. An automated laboratory system comprising the data processing system according to the preceding claim.

13. A computer program product comprising instructions which, when the program is executed by a computer, cause the computer to carry out the method according to any one of claims 1 to 10.

14. A computer-readable medium comprising instructions which, when executed by a computer, cause the computer to carry out the method according to any one of claims 1 to 10.
